# EUROPEAN PATENT APPLICATION

(11) **EP 1 865 000 A1**
(43) Date of publication of application: **12.12.2007**
(21) Application number: 06730069.9
(22) Date of filing: 27.03.2006
(51) Int. Cl.: C07K 16/18, G01N 33/577, G01N 33/58, C12N 15/00, C07K 19/00

(54) **MONOCLONAL ANTIBODY SPECIFIC TO DENATURED HUMAN LEUKOCYTE ANTIGEN CLASS I**

(30) Priority: 29.03.2005 JP 2005094920
(71) Applicant: Japan Science and Technology Agency, Kawaguchi-shi, Saitama 332-0012 (JP); Sato, Noriyuki, Sapporo-shi, Hokkaido, 0620042 (JP)
(72) Inventor: TORIGOE, Toshihiko, poro-shi, Hokkaido, 0640821 (JP); SHIMOZAWA, Kumiko, apporo-shi, Hokkaido, 0640807 (JP); NAKAZAWA, Emiri, ku, Sapporo-shi Hokkaido, 0040002 (JP)
(74) Representative: Wilson Gunn
(86) International application number: PCT/JP2006/306121
(87) International publication number: WO 2006/104085

(57) **Abstract**

[PROBLEMS] To provide a monoclonal antibody simultaneously detectable the expression of all three kinds of HLA-A, HLA-B and HLA-C heavy chain proteins composing human class I leukocyte antigens (HLA class I) in denatured human tissue samples fixed by formalin and the like.

[MEANS FOR SOLVING THE PROBLEMS] It was discovered that injection of denatured recombinant HLA-A*2402 heavy chain proteins to mouse for immunization led to establish HLA class I-specific antibody binding all of denatured HLA-A, B and C. The present invention is a monoclonal antibody specifically binding HLA-A, HLA-B and HLA-C heavy chains composing denatured human class I antigens, wherein said monoclonal antibody is produced by hybridomas (FERM AP-20454) and bound specifically HLA-A, HLA-B and HLA-C heavy chains composing denatured human class I antigens. Furthermore, the present invention is a test reagent for examining denatured human class I leukocyte antigens containing the monoclonal antibody as a major component.

SEQUENCE LISTING

<110>  Japan Science and Technology Agency

<120>  Monoclonal antibodies specific to denatured human class I leucocyte antigens

<130>  FS06-454PCT

<160>  1

<170>  PatentIn version 3.1

<210>  1
<211>  897
<212>  DNA
<213>  Homo sapiens

<400>  1
ggctcccact ccatgaggta tttctccaca tccgtgtccc ggcccggccg cggggagccc      60

cgcttcatcg ccgtgggcta cgtggacgac acgcagttcg tgcggttcga cagcgacgcc     120

gcgagccaga ggatggagcc gcgggcgccg tggatagagc aggaggggcc ggagtattgg     180

gacgaggaga cagggaaagt gaaggcccac tcacagactg accgagagaa cctgcggatc     240

gcgctccgct actacaacca gagcgaggcc ggttctcaca ccctccagat gatgtttggc     300

tgcgacgtgg ggtcggacgg gcgcttcctc cgcgggtacc accagtacgc ctacgacggc     360

aaggattaca tcgccctgaa agaggacctg cgctcttgga ccgcggcgga catggcggct     420

cagatcacca agcgcaagtg ggaggcggcc catgtggcgg agcagcagag agcctacctg     480

gagggcacgt gcgtggacgg gctccgcaga tacctggaga acgggaagga gacgctgcag 540

cgcacggacc cccccaagac acatatgacc caccacccca tctctgacca tgaggccact 600

ctgagatgct gggccctggg cttctaccct gcggagatca cactgacctg gcagcgggat 660

ggggaggacc agacccagga cacggagctt gtggagacca ggcctgcagg ggatggaacc 720

ttccagaagt gggcagctgt ggtggtacct tctggagagg agcagagata cacctgccat 780

gtgcagcatg agggtctgcc caagcccctc accctgagat gggagccagg atccctgcat 840

catattctgg atgcacagaa aatggtgtgg aatcatcgtc tggttccgcg tggttcc 897

## Description

### Field of the Invention:

The present invention relates to a monoclonal antibody specifically binding HLA-A, HLA-B and HLA-C heavy chains of denatured human class I leukocyte antigens, furthermore, to a method for examining denatured human class I leukocyte antigens by the use of the antibody, and to a test reagent for examining denatured human class I leukocyte antigens containing the antibody.

### Prior Art:

Most of the clinical tissue samples in the world have been preserved by 10% to 20% formalin fixative and the like for several decades. However, the fixative as formalin drastically denaturalizes proteins in tissues. Therefore, detection of proteins in fixed tissue samples by specific antibodies necessitates antibodies recognizing denatured proteins.
On the other hand, human class I leukocyte antigens (HLA class I) ares important molecules representing antigen molecules to immune-competent cells. For example, decomposition product-antigen peptides of virus proteins in virus infected cells and those of tumor antigen proteins in tumor cells independently bound to HLA class I molecules and are exposed to cell surface of respective cells. T cells among various immune-competent cells recognize antigen peptide-HLA complex on target cell surface by the use of T cell antigen receptors located on the cell surface, and thereby differentiate virus infected cells and tumor cells from normal cells. Therefore, suppressed expression of HLA class I molecules impairs the normal function in T cells to discriminate target cells and results in missing of virus-infected cells and tumor cells from immunological surveillance. Therefore, HLA class I antigen molecules are molecules playing important roles in immune systems and examination of the expression of the molecules in human tissues and cells gives important information for understanding immunological pathology in various human disorder.
In spite of the importance, conventional human class I leukocyte antigen (HLA class I) antibodies (e.g. W6/32 antibody) cannot recognize denatured HLA class I proteins and could not detect antigen proteins in tissues fixed by formalin and the like by an immunological staining method.

Meanwhile, two kinds of mouse monoclonal antibodies, whose clone names are HC10 and HCA2, are reported as monoclonal antibodies preferentially binding denatured HLA class I proteins (Reference 1). However, HC10 binds to HLA class I heavy chain proteins derived from two kinds of genes, such as HLA-B and HLA-C, but not to those derived from HLA-A gene. Furthermore, HCA2 binds to HLA class I heavy chain proteins derived from HLA-A gene, but not to those derived from HLA-B and HLA-C genes.
As explained above, the monoclonal antibodies, which are simultaneously detectable all of the expressed three kinds of HLA class I heavy chain proteins derived from HLA-A, HLA-B and HLA-C genes in denatured human tissues like samples fixed by formalin and the like, have not been reported previously.

Reference 1: Stam NJ, et al., Int Immunol. 1990; 2(2): 113-25.

### Problems to be solved by the Invention:

Human pathological tissue samples fixed by formalin and the like and preserved contain not only denatured HLA class 1 molecules but also denatured all kinds of tissue proteins. Therefore, in order to detect proteins in fixed tissues by monoclonal antibodies, it needs to use antibodies recognizable denatured proteins. But monoclonal antibodies recognizable all of HLA-A, B and C have not been present currently and are required.

### Means of Solving the Problems:

The present inventors discovered that injection of denatured recombinant HLA-A*2402 heavy chain proteins to mouse for immunization led to establish HLA class I specific antibodies preferentially binding all of denatured HLA-A, B and C and accomplished the present invention.
Namely, the present invention is a monoclonal antibody that specifically binds HLA-A, HLA-B and HLA-C heavy chains of denatured human class I leukocyte antigens.
Additionally, the present invention is a hybridoma producing the monoclonal antibody.
Also, the present invention is a monoclonal antibody binding specifically to HLA-A, HLA-B and HLA-C heavy chains of denatured human class I leukocyte antigen, which is produced by the hybridoma (FERM AP-20454).
Furthermore, the present invention is immunogloblin gene information of said hybridomas and recombinant proteins produced based on the gene information.
Moreover, the present invention is a method for examining denatured human class I leukocyte antigen comprising reacting the monoclonal antibody with denatured test samples of human-derived cells or tissues
Still furthermore, the present invention is a test reagent for examining denatured human class I leukocyte antigens comprising the monoclonal antibody as a major component.

### Brief Description of the Drawings

Figure 1 shows an example of immunostaining of paraffin embedded slices of colorectal cancer tissue fixed by formalin. Figure 1 (1) shows HLA class I positive example in a tumor tissue. Figure 1 (2) shows HLA class I negative example in a tumor tissue.
Figure 2 shows reactivity test result between EMR8-5 antibodies and various recombinant HLA class I allele proteins by Western blotting. EMR8-5 antibodies react with all of recombinant proteins derived from HLA-A, HLA-B and HLA-C alleles, while HC10 antibodies do not react with HLA-A allele-derived proteins.
Figure 3 shows an example of reaction of EMR8-5 antibodies with various human tumor cell lysates by Western blotting. EMR8-5 reacts with HLA class I heavy chains expressed in oral cavity carcinoma cell line OSC20 and OSC20-A2402 cells transduced with HLA-A2402 gene. The antibodies do not react with cell lysates from HLA class I negative cell line K562. The right lane shows the reactivity with recombinant HLA-A2402 heavy chain proteins used as immunogens of positive control.
Figure 4 shows the relation between the results of immunostaining of formalin fixed kidney cancer tissues removed during operation in the past and the survival rate of the patients.

### Effects of the Invention

The monoclonal antibody of the present invention enabled to perform immunological tissue staining of heavy chain proteins derived from HLA-A, B, C in paraffin embedded section fixed by formalin and the like. The method enabled not only to detect HLA class I antigens by the use of pathologic tissue samples fixed by formalin and the like submitted as surgically isolated specimens or biopsy samples in daily clinical situation, but also to search retrieved HLA class I antigens in formalin-fixed paraffin embedded samples under preservation.
Still moreover, as shown in example 4 as described above, examination of HLA class I in cancer tissues is useful for prognostic diagnosis of patients as well as pathogenic diagnosis of cancer.
Also, as shown in example 5 as described above, the immunostaining method of cancer tissue by the use of the present antibodies is useful as a diagnostic method to determine the applicability of an immunotherapy depending on CTL.

### The best mode to perform the Invention

Human class I leukocyte antigens (HLA class I) are mainly composed of heavy chains encoded by 3 kinds of genes, i.e. HLA-A, HLA-B, HLA-C, and a heterodimer by two light chain molecules encoded by a gene referred to as beta 2-microglobulin. There exists gene polymorphism for the genes of heavy chains. For example, it is known that the most frequent HLA gene in Japanese is the gene (Genbank ACCESSION #M64740) referred to as HLA-A*2402.

The mouse monoclonal antibody of the present invention binding preferentially to denatured human class I leukocyte antigen (HLA-A, B, C) heavy chains are antibodies produced in the culture supernatant of the hybridomas (the clone name is EMR8) and the subclone, and mouse monoclonal antibody carrying subclass IgG1, κ chain. The antibodies are referred to as EMR8 antibodies.
Hybridomas EMR8 were deposited on March 9, 2005 to International Patent Organism Depository, Advanced Industrial Science and Technology as accession number FERM AP-20454 , transferred to International Authority Depository, and provided accession number FERM BP-10550 from the depository on March 9, 2006. The denaturation method involves treatment with aldehydes such as formalin, paraformaldehyde, glutaraldehyde and the like, alcohol, acetone, urea, guanidine hydrochloride, formic acid, heat treatment and the like, and preferably, treatment with aldehyde, acetone and alcohol.

The antibodies of the present invention are produced in the culture supernatant of hybridomas EMR8 and the subclones. The culture supernatant may be used for detection of HLA class I antigens by itself. Furthermore, the hybridomas could be transplanted into mouse peritoneal cavity and the ascites fluid containing EMR8 antibody may be used for the detection. Moreover, immunoglobulin gene DNA or RNA could be isolated from hybridoma EMR8 cells and recombinant proteins produced by recombinant gene containing a variable region of the gene sequence may be used.
The antibodies of the present invention enable to detect denatured human class I leukocyte antigen (HLA-A, B, C) heavy chains expressed in tissues in paraffin-embedded section fixed by formalin and the like by the use of immunostaining or Western blotting. Therefore, the antibodies of the present invention may be used as a reagent for clinical examination, tissue staining and detection of HLA class I.
For example, the antibodies could be applied to expression test of HLA, class I antigen proteins in tissues of human cancer and virus infection fixed by chemicals such as formalin and paraformaldehyde, expression level analysis of HLA class I antigen proteins in pathological tissues of various human disease and detection of intra cellular localization of the proteins.

When the antibodies of the present invention are used as a reagent to detect HLA, the antibodies of the present invention could be used by themselves or together with other antibodies. Furthermore, the antibodies could be labeled directly with a fluorescent dye or labeled with an enzyme.
There are no restrictions on the method to detect the antigen antibody reaction, but the method involves immunoblot, dot blot and ELISA, preferably ELISA.
As an example of the test method, the antibodies of the present invention are reacted with HLA in a test sample, followed by the reaction with a probe recognizing the antibodies. The probe involves anti-human IgG antibodies, protein G, protein A and protein L. The probe is labeled with an indicator. The indicator involves radioactive isotope (¹²⁵I), enzymes (peroxidase, alkaline phosphatase), fluorescent compound, light-emitting material and the like. The use of enzyme-linked antibodies only needs reaction of the antibodies with a substrate for observation of the reaction (color change)
The following examples are provided to illustrate the present invention, but are not intended to limit the scope thereof.

### Example 1

cDNA (SEQ ID NO: 1) coding extra cellular domain of HLA-A*2402 heavy chain protein was inserted into E. coli expression vector and histidine tag-fused recombinant HLA-A*2402 heavy chain proteins were prepared.
The cDNA (SEQ ID NO: 1) contains a structure with two gene sequences, which code BirA substrate peptide and thrombin recognizing peptide respectively, binding 3' end of bases 73-900 of HLA-A2402 cDNA (Genbank ACCESSION #M64740). The gene codes a fused protein composed of biotinized domain and thrombin cleavable domain at the C terminal of extra cellular domain of HLA-A2402 heavy chain protein without containing a signal sequence (Journal of Immunological Methods 271, 177-184, 2002).
E. coli cells were disrupted by sonication, dissolved in urea-HEPES buffer, and charged to nickel NTA agarose column bounded with His tag-recombinant proteins, followed by elution with imidazole added-urea HEPES buffer. The solution was dialyzed against PBS for over night to give denatured and aggregated recombinant HLA-A*2402 heavy chain protein suspension.

The emulsion prepared by the heavy chain protein suspension (about 1 mg equivalent recombinant proteins) and complete Freund's adjuvant was injected to BALB/c mice subcutaneously. After the second injection, suspension of incomplete Freund's adjuvant was injected every two weeks for eight times. At 5 days after the final injection, a spleen of the mice was isolated and spleen cells were fused with mouse myeloma cell line NS-1. The hybrid cells were cultured in HAT selection medium for about a month and about 100 hybridoma colonies were obtained. The culture supernatant was recovered and subjected to the first screening.
Hybridomas reacted with urea-denatured recombinant HLA-A*2402 heavy chain proteins were selected by ELISA as the first screening. More precisely, an ELISA plate was covered with solidified urea-denatured recombinant HLA-A*2402 heavy chain proteins and added with hybridoma culture supernatant. After 2 hrs, the plate was washed, reacted with peroxidase-labeled anti-mouse immunoglobulin, washed, and colored by peroxidase substrate solution. The second screening was performed for about 15 positive hybridoma clones obtained.

The second screening was performed by immunostaining of human pathological tissues in paraffin embedded section fixed by formalin. Each HLA positive human cancer tissue slice sample was added with 15 kinds of hybridoma culture supernatant. After washing, the samples were reacted with peroxidase-labeled anti-mouse immunoglobulin antibody, washed and colored by peroxidase substrate solution. Positive control staining was performed by anti-HLA-B, C antibody HC10. Those samples with colored cell membranes as HC10 were judged as positive. As the result, a kind of hybridomas was positive.

The third screening was performed by Western blotting. Respective cell lysates were prepared for HLA positive human cell line OSC-20, HLA-A*2402 gene-transformed cell line OSC-20A24, HLA class I negative cell line K562. After SDS electrophoresis, the sample was transferred to a PVDF membrane. The protein-transferred membrane was added with hybridoma culture supernatant. After washing, the membrane was reacted with peroxidase-labeled anti-mouse immunoglobulin antibodies. After washing, the membrane was colored by peroxidase substrate solution ECL. Anti-HLA-B and C antibodies HC10 used as positive control were immunoblotted. Recombinant HLA-A*2402 heavy chain proteins used as positive control antigens were electrophoresed. The positive hybridomas were selected based on the specific bands detected as HLA class I heavy chains in recombinant HLA-A*2402 heavy chain proteins and OSC-20 cell lysates.
The above three-step screening led to select positive hybridomas ERM8 (FERM AP-20454).

The hybridomas were cloned by limiting dilution to give 20 subclones. The subclones were numbered as EMRB-1 to EMR8-20. The culture supernatant of these subclones were assayed by the methods of the above second and third screening and were confirmed to recognize specifically denatured HLA class I heavy chain proteins.
Furthermore, the reaction specificity of EMR8 subclones was analyzed by Western blotting of recombinant heavy chain proteins derived from HLA-A, B and C genes with various gene polymorphism. It was shown, as the result, that EMR8 subclones reacted with all the HLA-A, B and C heavy chains examined, while positive control antibodies HC 10 reacted with only HLA-B and C heavy chain. EMR8 antibodies are mouse monoclonal antibodies with subclass IgG1, κ chains and could simultaneously detect HLA-A, B and C expressed in cell membranes of pathological tissues in paraffin embedded slices fixed by formalin.
The positive control antibodies HC10 were provided by Dr. Soldano Ferrone (Department of Immunology, Roswell Park Cancer Institute, Buffalo, New York).

### Example 2

In this example, immunostaining of formalin fixed human tissue by the use of the antibodies of example 1. The procedures are as follows:
(1) Paraffin embedded slices of human colorectal cancer tissue fixed by 20% formalin fixative was treated with ethanol to remove paraffin.
(2) The slices were soaked in 0.01 mol/L citric acid buffer (pH 6.0) and treated with microwave (95° C, 15 min) as antigen activation treatment.
(3) 0.5 ml of ten times diluted solution of hybridoma EMR8-5 culture supernatant was dropped on respective slices and incubated at room temperature for 1 hr.
(4) The slices were washed with washing solution PBS-T (0.05% Tween20/PBS, pH 7.4) for three times.
(5) Peroxidase labeled anti-mouse IgG antibodies (Simple stain MAX-PO, NICHIREI), the second antibodies, were dropped on respective slices and incubated at room temperature for 30 min.
(6) The slices were washed with washing solution PBS-T (0.05% Tween20/PBS, pH 7.4) for three times.
(7) The slices were soaked into the mixture solution (Simple stain MAX-PO, NICHIREI) of hydrogen peroxide and DAB substrate and left for colored reaction for 1 to 2 min.
(8) The slices were washed with running water for 1 min.
(9) The slices were colored with Hematoxylin nuclear staining (1 to 2 min).

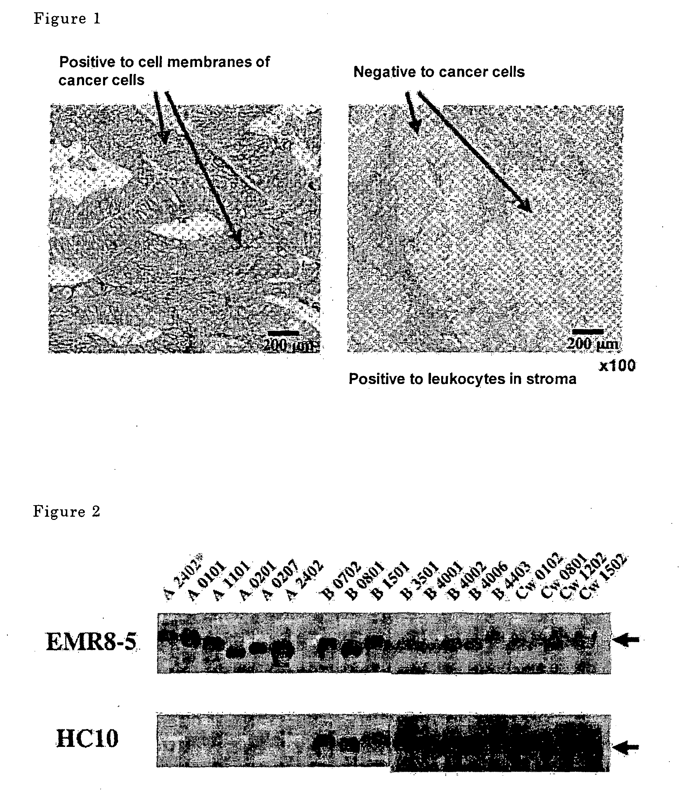

Figure 1 shows the example of immunostaining of paraffin embedded slice of fixed colorectal cancer tissue by formalin. Fig. 1(1) shows stained HLA class I as brown in the cancer tissue, while Figure 1(2) shows negatively colored HLA class I in tumor cells and shows brown staining in lymphocytes invaded into tumor stroma and endothelial cells. More precisely, there are HLA class I antigen positive and negative colorectal cancers, and it is demonstrated that these cancer tissues are discernible by immunostaining method by the use of EMR8 antibodies as shown in example 2.

### Example 3

In the example, Western blotting was performed by the use of the antibodies of the present invention. The procedures are as follows:
(1) 1x10⁶ cells were lysed in 100 µl cell lysis solution (RIPA buffer) and soluble fraction was recovered as lysate. The lysate was added with SDS sample buffer.
   Recombinant proteins used were HLA heavy chain recombinant proteins provided from Medical & Biological Laboratory, CO., LTD. Note that HLA-A2402* is His-tag fused recombinant proteins obtained in example 1.
(2) The recombinant proteins dissolved in 6 M urea buffer were added with SDS sample buffer.
(3) The protein samples were loaded to 7.5% SDS polyacrylamide gel and electrophoresed.
(4) Proteins in gel were transferred to PVDF membrane.
(5) The transferred membrane was soaked into 5% skim milk· PBS for about 1 hr for blocking.
(6) The transferred membrane was soaked into the first antibodies, ten times diluted solution of hybridoma EMR8-5 culture supernatant, and incubated at room temperature for 1 hr.
(7) The transferred membrane was washed with washing solution PBS-T (0.05% Tween 20/PBS, pH 7.4) for three times.
(8) The transferred membrane was soaked into the second antibodies, peroxidase labeled anti-mouse IgG antibody, and incubated at room temperature for 1 hr.
(9) The transferred membrane was washed with washing solution PBS-T (0.05% Tween 20/PBS, pH 7.4) for three times.
(10) The transferred membrane was soaked into ECL kit (Amersham, USA), illumination solution, for 1 min for illuminating reaction.
(11) The illumination signal was detected by X-ray film.

Figure 2 shows the result of reaction test between EMR8 antibodies and various recombinant HLA class I allele proteins by Western blotting.
EMR8 antibodies reacted with all of the HLA-A, B and C allele-derived recombinant proteins shown in the figure, while positive control antibodies HC 10 do not react with HLA-A allele-derived protein. More precisely, it is shown that EMR8 antibodies unlike with HC10 antibodies recognize all three HLA-A, B and C allele-derived HLA class I heavy chain proteins in Western blotting.

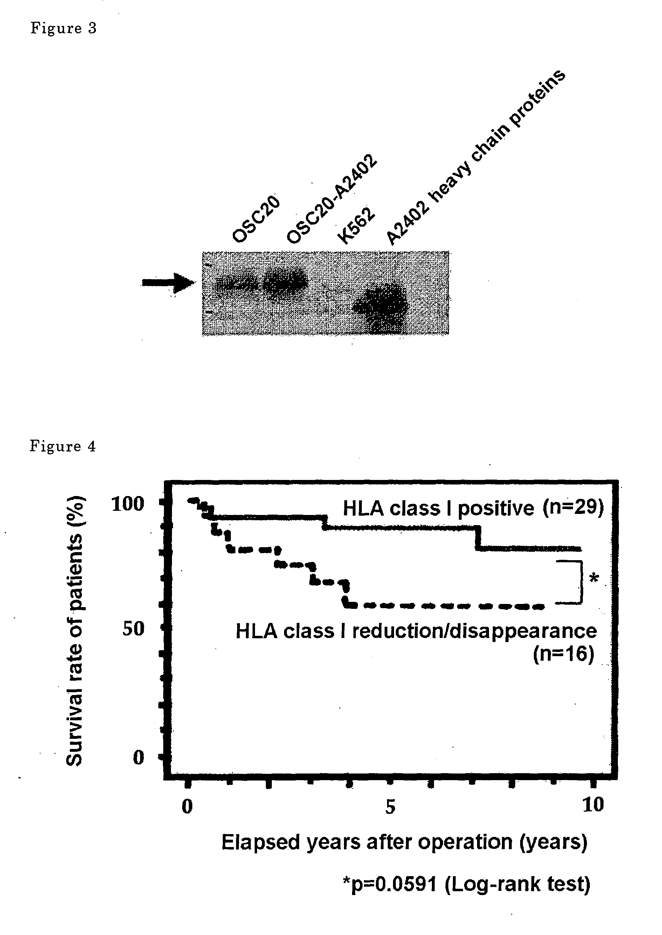

Figure 3 shows the reaction example between EMR8 antibodies and human various tumor cell-lysates by Western blotting. The cells used involve three kinds of cell lines, i.e. (i) HLA-A24 negative human oral cavity carcinoma cell line OSC20, (ii) OSC20-A2402 obtained by transduction of HLA-A*2402 gene into OSC20 cells and stably expressing HLA-A24, and (iii) human leukemia cell line K562 without expressing all HLA class I genes.
EMR8 antibodies react with HLA class I heavy chains expressed in oral cavity carcinoma cell line OSC20 and OSC20-A2402 cell line transduced with HLA-A*2402 gene, but not with lysates of HLA class I negative cell line K562. The right lane shows the reaction with recombinant HLA-A*2402 heavy chain proteins, i.e. positive control antigens. It is shown by Western blotting that EMR8 antibodies recognize not only recombinant HLA class I heavy chain proteins but also HLA class I heavy chain proteins intra-cellularly expressed.

### Example 4

Formalin fixed renal cancer tissue samples of 45 cases removed by operation in the past was immunostained by the similar method to example 2 by the use of EMR8 antibodies of the present invention and examined the relation to their survival rate.
As shown the result in Fig.4, 64% cases are HLA class I expression positive, while other 36% cases are reduced in expression of HLA class I. Comparison of patient survival rate between positive and negative groups after operation revealed that the survival rate of HLA reduced group is significantly lower than that of increased group. Thus, HLA class I test of cancer tissues is useful not only for pathological diagnosis of cancer but also for prognostic diagnosis of patients.

### Example 5

Development of immunotherapy, which involves cancer vaccination therapy, dendritic cell therapy and genetic immunization therapy aiming for induction of cytotoxic T cell (CTL) specific to cancer cells by the use of antigen peptide presented by HLA class I molecules, has been performed through the world. It is important as an adaptability toward the therapy to test whether HLA class I antigens are expressed on cell surface. In spite of this, since antibodies reactive to all of HLA-A, B and C and usable to immunostain formalin fixed samples similar to the antibodies of the present invention has not been present up to now, tests for immunotherapy have been performed without checking whether cancer tissue of a patient is HLA class I antigen positive or negative. The present inventors has performed clinical test of cancer peptide vaccination by the use of antigen peptide Survivin 2B peptide (Japanese Patent Application Public Disclosure No. 2002-284797) derived from cancer antigen, Survivn, since 2003.

On 15 cases of clinical trials for colorectal cancer, the correlation between the clinical results (tumor shrinkage or increase attenuation effect) by the above method and immunostaining results of HLA class I antigens by the use of the present antibodies in cancer tissues before vaccination was examined. The tissue samples used were treated with formalin and the test method was according to the method described in example 2 in the test by the use of the antibodies of the present invention.
According to the results, all five cases with positive clinical effect showed strongly positive results for immunostaining of HLA class I antigens by EMR8 antibodies, while four cases out of ten cases without clinical effect showed disappearance or decrease of HLA class I antigens. Therefore, positive correlation was confirmed between clinical effect of cancer immunotherapy and expression level of HLA class I antigens.
More precisely, the cases, wherein HLA class I antigens are positively stained in cell membranes of cancer cells by immunostaining of cancer tissues by the use of EMR8 antibodies, have expected positive effect of CTL inducible immunotherapy. Therefore, the immunostaining is useful as decision-making tool for adaptability to cancer immunotherapy.

## Claims

1. A monoclonal antibody that specifically binds HLA-A, HLA-B and HLA-C heavy chains of denatured human class I leukocyte antigens.

2. A hybridoma producing the monoclonal antibody of claim 1.

3. The hybridoma of claim 2, wherein said hybridoma is a deposited cell (FERM AP-20454).

4. A monoclonal antibody binding specifically to HLA-A, HLA-B and HLA-C heavy chains of denatured human class I leukocyte antigen, which is produced by the hybridoma (FERM AP-20454).

5. The monoclonal antibody of claim 1 or 4, wherein said denatured human class I leukocyte antigen is obtained from human tissues treated with aldehyde , alcohol or acetone.

6. A method for examining denatured human class I leukocyte antigen comprising reacting the monoclonal antibody of claim 1 or 4 with test samples.

7. The method of claim 6, wherein said samples are denatured samples of human-derived cells or tissues

8. The method of claim 7, wherein said denaturation is performed by treatment with aldehyde, alcohol or acetone.

9. The method of claim 6 comprising reacting said samples with said monoclonal antibody, then reacting with labeled probe and detecting the label.

10. A test reagent for examining denatured human class I leukocyte antigens comprising the monoclonal antibody of claim 1 or 4 as a major component.

11. Immunoglobulin genetic information of the hybridomas of claim 2 or 3.

12. A recombinant protein prepared based on the genetic information of claim 11.
